# EUROPEAN PATENT SPECIFICATION

(11) **EP 1 520 139 B1**
(45) Date of publication and mention of the grant of the patent: **01.08.2012**
(21) Application number: 03704984.8
(22) Date of filing: 10.02.2003
(51) Int. Cl.: B01L 7/00, C12M 1/18, F25D 3/02, F25D 25/00, F26B 5/06, A01N 1/02

(54) **CHANGING THE TEMPERATURE OF A LIQUID SAMPLE AND A RECEPTACLE USEFUL THEREFOR**
VERÄNDERN DER TEMPERATUR EINER FLÜSSIGEN PROBE UND BEHÄLTER HIERFÜR
CHANGEMENT DE LA TEMPERATURE D'UN ECHANTILLON LIQUIDE ET RECEPTACLE UTILISE A CET EFFET

(30) Priority: 27.06.2002 US 391575 P; 10.10.2002 US 417460 P
(43) Date of publication of application: 06.04.2005
(73) Proprietor: Core Dynamics Limited, Hamilton HM EX (BM)
(72) Inventor: ARAV, Amir, 62266 Tel Aviv (IL)
(74) Representative: Brophy, David Timothy
(86) International application number: PCT/IL2003/000101
(87) International publication number: WO 2004/003444

(56) References cited:
- FR-A- 2 574 919
- US-A- 5 873 254

## Description

### FIELD OF THE INVENTION

This invention relates to a method and apparatus for changing the temperature of a liquid sample, particularly the freezing and thawing of biological samples such as semen, and a receptacle useful therefor.

### BACKGROUND OF THE INVENTION

Cryopreservation of cells, tissues and organs has vast implications on numerous procedures, for example grafting, *in vitro* manipulation (such as *in vitro* fertilization), research, etc. In a conventional slow-freezing method used for biological samples, a chamber is used in which the sample is introduced for freezing. Then, the temperature of the chamber is dropped in a controlled stepwise manner, thus exposing the sample to an external and gradual change in temperature.

A different technology for freezing is the "Multi-temperature gradient" (MTG) directional solidification, which is based on the invention disclosed in US 5,873,254. In this technology, the sample is moved at a constant velocity (V) through temperature gradients (G) so the cooling rate (G x V) and ice front propagation are controlled and the velocity of the movement of the sample determines the morphology of the ice crystals formed within the sample. This method also enables the incorporation of controlled seeding into the freezing process.

The freezing of samples according to any of the known methods, even when using accurate freezing rate control systems, is typically adapted for small samples that are 5 milliliters or less in volume. This is partially due to the fact that, in large samples, some parts of the sample (usually the outer zone or part thereof) may chill or warm faster than other parts. Thus, freezing and storage of semen is performed regularly using mini (¼ cc) or midi (½ cc) straws. Samples with volumes of nearly 5 milliliters are usually frozen in plastic bags that are flattened during the preservation process, so as to have at least one dimension of the sample not exceeding 0.5 cm.

Solutions enabling freezing of larger samples have been suggested in co-pending PCT/IL02/00738 and PCT/IL03/00026. In the former application, a method is described wherein the sample is agitated during freezing under the directional solidification process. Thus, the rate of heat transfer within the sample is amplified, and the effect of the sample's bulk, morphology and heat transfer rate on the morphology of the forming ice crystals is reduced. In the latter application, an "isothermal-break" method is disclosed. In this method, the freezing or thawing of a sample is performed such that the sample is kept at a desired intermediate temperature at least for a time that would allow the temperature of the sample or a section thereof to become uniform and equal to the intermediate temperature.

A receptacle according to the preamble of claim 1 is known frorm FR-A-2574919

### SUMMARY OF THE INVENTION

The term *"biological sample"* means, in the present description and claims, an amount of biological matter including cells and/or group of cells and/or bodily fluids and/or any constituents thereof. For example, a sample may comprise semen, oocytes (ova), blood, blood cells, blood constituents, germ cells, umbilical cord blood, plasma, zygotes, embryos, etc.

The term *"liquid sample"* means, in the present description and claims, any sample which is essentially liquid but which may contain cells, cell parts, biological macromolecules, complexes of various substances, liposomes etc. Such sample may be derived from or contain a biological fluid (e.g. blood, semen, etc.). It may contain, in addition to or instead of the bodily fluid, also a non-biological liquid (e.g. a chemical solution). In fact, the term "liquid sample" is not limited to a biological sample and may comprise or consist of non-biological matter, for example, sensitive synthetic polymers.

One aspect of the present invention is connected with the use of a receptacle designed such that the surface/volume relation in a sample carried thereby is increased, whereby its ability to be uniformly frozen may be highly facilitated. This is achieved by an annular shape of the receptacle and, consequently, of the sample carried thereby, due to which the sample is free of a central core, the temperature of which would be the last to change especially if the sample has a large diameter. The annular receptacle may have other benefits in the cooling and warming of a liquid sample. One of which is that it may be cooled/or warmed both from the outside in and from the inside out.

The receptacle of the present invention is defined in claim 1. It has proximal and distal ends, an inner wall and an outer wall defining an annular portion therebetween adapted for receiving liquid therein, and an inner space defined by said inner wall. The inner wall 15 open at each of said proximal and distal ends, enabling passage of fluid via said inner space whilst holding said liquid within said annular portion.

According to another aspect of the present invention, there is further provided a method for changing the temperature of a liquid sample, comprising:
(i) providing a receptacle according to claim 1 ,
(ii) inserting said liquid sample, at a first temperature, into said annular portion, and
(iii) exposing said receptacle to a second temperature different from said first temperature.

When a biological sample containing living cells in a freezing solution is frozen, the first portion of the sample to freeze is the intercellular fluid. The formation of ice in the intercellular fluid increases the salt concentration therein. If the sample is frozen too slowly, the high concentration of salt in the intercellular fluid may kill the cells, by osmotic shock or by chemical toxicity. Conversely, freezing the sample too rapidly may lead to the formation of intracellular ice crystals, which also kill the cell, by internal mechanical damage. In addition, the rate of cooling affects the morphology of the intercellular ice crystals. Morphologies such as closely packed needles also kill cells, by external mechanical damage. Thus, maximizing the survival rate of cells subjected to freezing and thawing requires careful control of the freezing process.

In essence, any change in the temperature of a biological sample may damage it. Sperm, for example, may die or be injured such that after thawing of a frozen sperm sample there less live sperm will be observed and the surviving sperm may be less viable and/or less fertile than before they were subjected to the temperature change. However, as long as the *"resultant quality"* of the sample is such that the biological sample remains useful for a given purpose (e.g. cell line propagation, fertilization, grafting, infection, research purposes, etc.), the freezing and/or thawing are considered successful. Obviously, the higher the survival rate and the less the damage, the better is the method for changing the sample's temperature. However, each biological sample (depending on its purpose and composition) would have a *"predetermined acceptable resultant quality"* which is the worst quality of the biological sample after its temperature has been changed, that would still allow the sample to be useful. For example, in equine sperm under given circumstances, 35% post-thaw progressive linear motility (PLM; discussed below) is commonly considered a "passing" result, whereas less than 35% is considered "failing". Accordingly, the *"predetermined acceptable resultant quality"* may be that a desired number of the sperm samples would display, after having been frozen and thawed, 35% and higher progressive linear motility.

Thus, according to yet another aspect of the present invention a receptacle according to claim 1 is provided for use in changing the temperature of a biological sample, said sample being characterized by a cross-sectional dimension along which the change of temperature may be performed with a predetermined acceptable resultant quality of the sample, said receptacle comprising an inner wall and an outer wall defining an annular portion therebetween for receiving said biological sample therein, said annular portion having a distance between said inner wall and said outer wall not exceeding said cross-sectional dimension of the sample.

In US 4,140,489 to Lee, there is described a test tube having an annular portion, between the outer wall and inner wall constituted by an inverted inner tube, which is blocked at one end. The test tube is used for viewing microbial colonies, where a dark background can be inserted within the cavity of the inner tube to increase contrast, which clearly has nothing in common with the idea of the present invention.

As mentioned above, one of the benefits of having a receptacle with an annular portion in accordance with the present invention, is that the temperature of a liquid sample in such receptacle may be changed not only from the outside, but also from the inside, i.e. via the inner space surrounded by the annular portion.

Accordingly, the present invention further provides a chamber containing the receptacle according to claim 1, said chamber facilitating changing the temperature of a liquid sample held within a receptacle,
said chamber comprising:
- a cavity for receiving said receptacle, and
- an inlet for inputting a heat transfer fluid into said cavity.

### BRIEF DESCRIPTION OF THE DRAWINGS

In order to understand the invention and to see how it may be carried out in practice, some embodiments will now be described, by way of non-limiting examples only, with reference to the accompanying drawings, in which:
**Fig. 1** is a perspective view of a receptacle and a plug according to one embodiment of the present invention;
**Fig. 2** is a perspective view of a receptacle and a plug according to another embodiment of the present invention;
**Fig. 3** is a perspective view of the receptacle of Fig. **1** with a conduit for facilitating the flow of a heat transfer fluid;
**Fig. 4A** is a partially cross-sectional view of a chamber according to the present invention with an inlet for facilitating the flow of a heat transfer fluid, and with a receptacle and plug as in Fig. **1** received therein;
**Fig. 4B** is a top view of the chamber of Fig. **4A**;
**Fig. 5** is a schematic illustration of one example of use of the chamber shown in Fig. **4A**;
**Fig. 6** is a cross-sectional view along the line A-A, of a receptacle of the present invention, similar to that shown in Fig. **1**, with the indication of examples of dimensions of such receptacle;
**Fig. 7A** is a graphical representation of the temperatures measured during freezing at an inner (mid-point) position and an outer (wall) position of a liquid sample in a conventional test tube;
**Fig. 7B** is a graphical representation of the temperatures measured during freezing at an inner (mid-point) position and an outer (wall) position of a liquid frozen in a receptacle such as shown in Fig. **1**;
**Fig. 8** is a graphical representation of the temperatures measured during thawing at an inner (mid-point) position of a conventional tube and the receptacle of Fig. **6**, with and without pumping water into the chamber during thawing;
**Fig. 9** is a column chart comparing the viability and motility parameters measured for equine semen after one of the following: (a) being chilled for 30 hours, (b) being frozen in a straw using a Planer freezing apparatus or (c) being frozen in a receptacle as shown in Fig. **6**;
**Fig. 10A** is a pie chart showing the rates of failing and passing in the progressive linear motility (PLM) tests for different stallions after having their sperm frozen in straws;
**Fig. 10B** is a pie chart showing the rates of failing and passing in the progressive linear motility (PLM) tests for different stallions after having their sperm frozen in receptacles as shown in Fig. **6**;

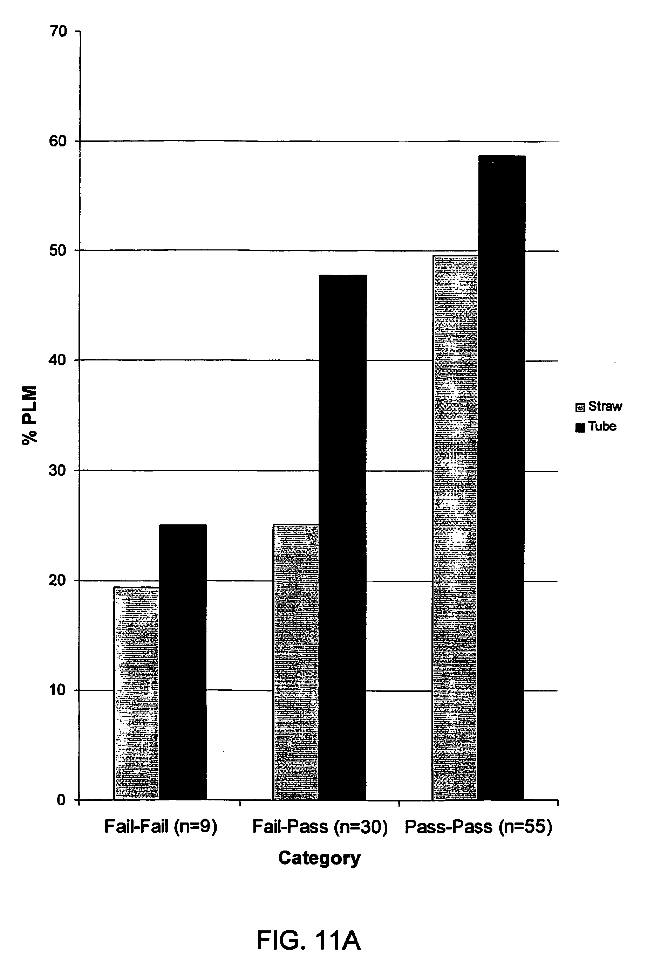

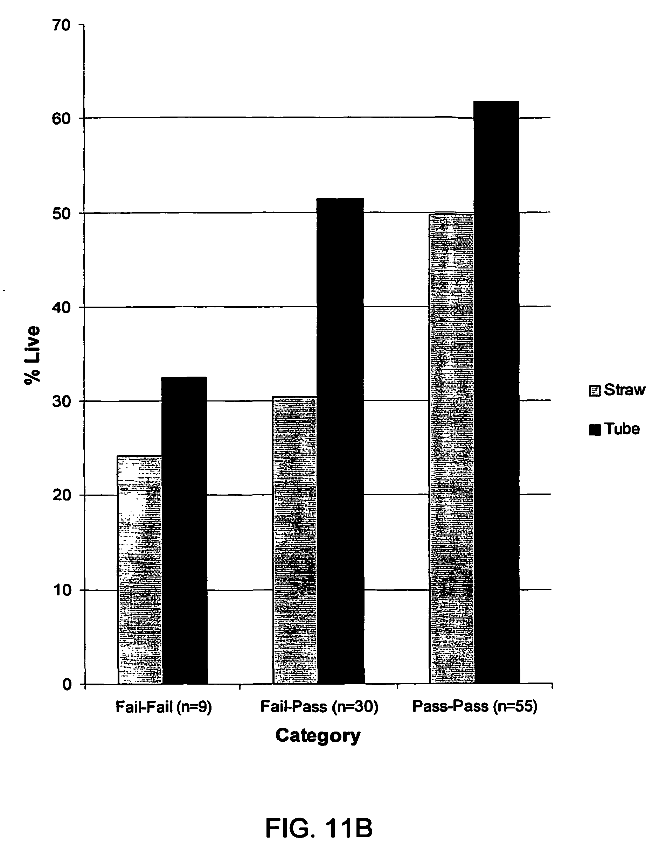

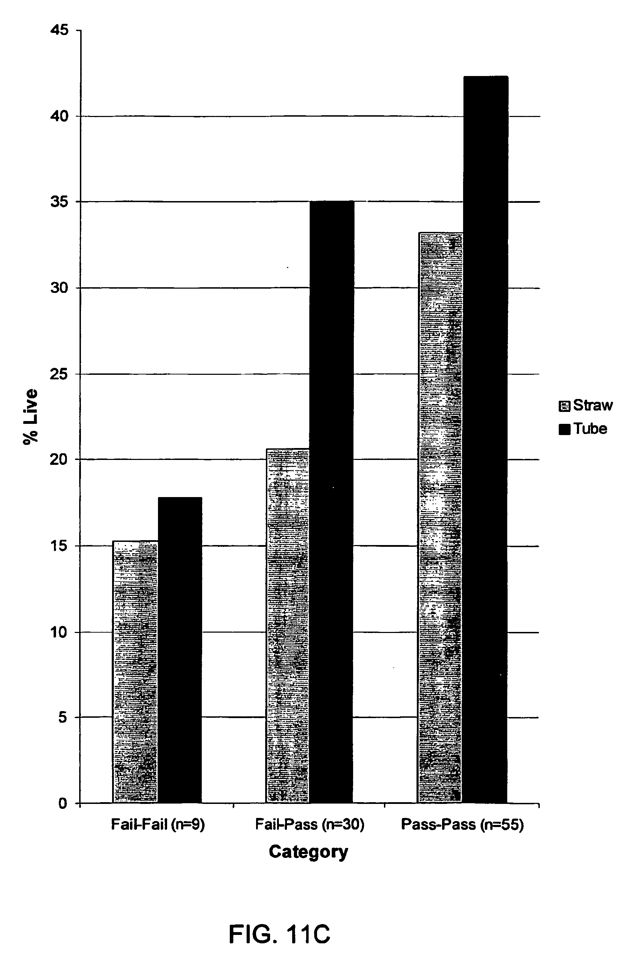

**Figs. 11** is a comparison of the post-thaw parameters measured for sperm that was frozen and thawed either in a straw or in a receptacle as shown in Fig. **6**. The results are grouped according to its failing or passing of the progressive linear motility (PLM) test after thawing where: **Fig. 11A** relates to the progressive linear motility of the sperm, **Fig. 11B** relates the percent of live cells according to an AO/PI assay, and **Fig. 11C** relates the percent of live cells according to an ORT assay.

### DETAILED DESCRIPTION OF THE INVENTION

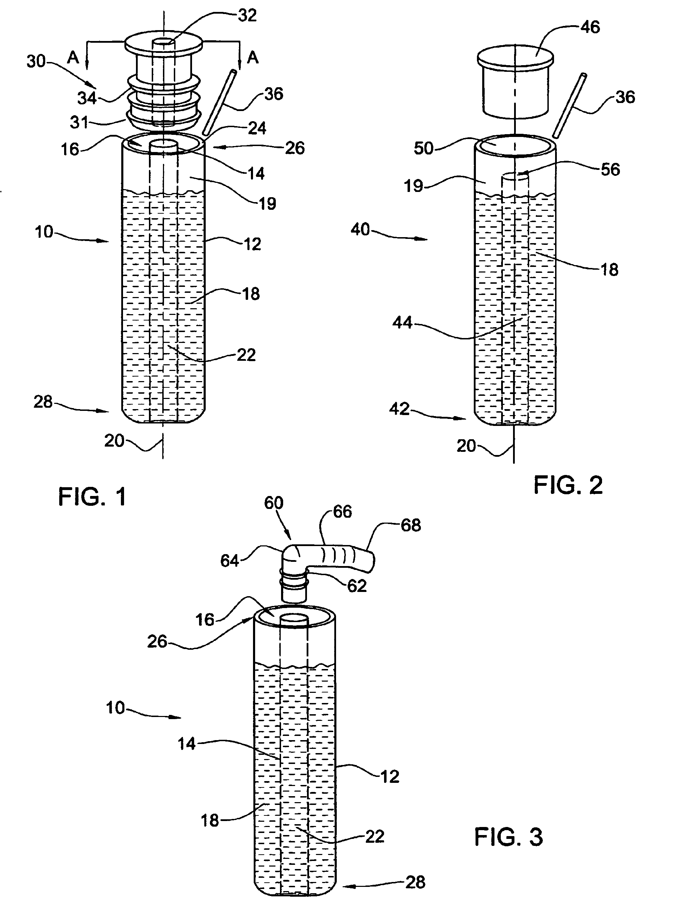

Referring first to Fig. **1** of the drawings, there is shown a test-tube like receptacle **10** comprising an outer wall **12** and an inner wall **14** defining therebetween an annular portion **16** within which a liquid **18** may be received. A longitudinal axis **20** extends through the center of an inner space **22** defined by the inner wall **14** of the receptacle **10.** The annular portion **16** has an opening **24** at a proximal end **26** of the receptacle **10** and is sealed at a distal end **28.**

It should be noted that the geometry of the receptacle **10** is advantageous in both the rate of heating/cooling of the liquid **18,** as well as the uniformity of the temperature during the heating/cooling process, in that it provides a reduced heat transfer path as compared to a receptacle such as a standard test tube of similar outer dimensions. The receptacle may be manufactured from any material that would allow heat transfer to and from the sample, and that would withstand the temperatures changes to which it is subjected, including glass, metal and various polymers, etc. The receptacle has an essentially circular cross-section taken perpendicularly to the longitudinal axis, however, the present invention is not limited to such cross-section. Other possible cross-sections may be square, rectangular, octagonal, and even irregular. Naturally, the stoppers used with the various shapes of cross-section would need to have a sealing portion compatible with that of the receptacle.

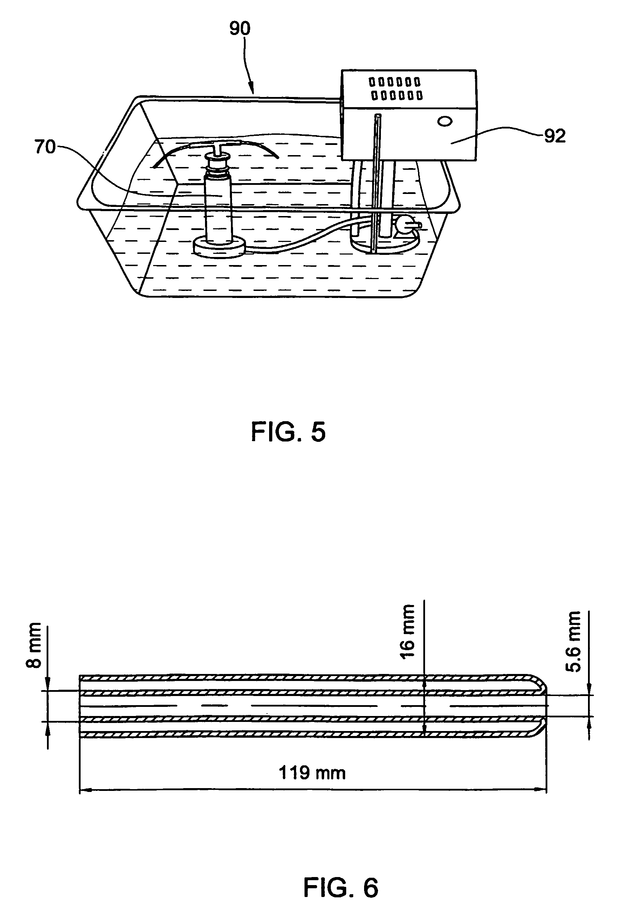

The outer and inner walls **12** and **14** provide increased heat transfer surface to the receptacle **10.** The inner space **22** is open at both the distal end **28** and proximal end **26** of the receptacle as shown in Fig. **6****,** such that a heat transfer fluid may flow into and through the inner space in from the distal end **28** and out via the proximal end **26,** or vice versa, without entering the annular portion **16.**

It is appreciated that in some cases it may be useful or necessary to seal the proximal end **26** of the receptacle **10,** so that matter may not exit and or enter the annular portion **16,** such as when contaminants are to be kept out, to protect a handler and surroundings, to prevent spillage and loss of possibly valuable liquid, etc.

One means for sealing the proximal end **26** of the annular portion **16** is a plug **30** (Fig. **1**), which has a bore **32** corresponding to and aligned with the inner space **22** of the receptacle **10.** Accordingly, the part of the plug **30** surrounding the bore **32** has an annular sealing portion **31** corresponding to the proximal end **26** of the annular portion **16.** The configuration of plug **30** is useful especially when heat transfer fluid, used to warm or cool the liquid sample, is arranged to flow through inner space **22.** In such case the heat transfer fluid may flow in and out of the proximal end **26** of inner space **22** via bore **32** without entering the annular portion **16** wherein the liquid sample is located.

The plug **30** further comprises flexible ribs **34** for providing proper sealing of the receptacle **10 -** although the plug itself may be made of a flexible/resilient material for providing a proper seal without the need for ribs.

Further seen in Fig. **1** is a wand **36** associated with the plug **30.** Such wand **36** may be an integral, removable part of plug **30** or merely in close association thereto. Upon inserting the plug **30** into the receptacle **10,** the wand **36** is placed at the periphery of the plug. This provides an escape for air **19** from the annular portion **16** that would otherwise be compressed upon sealing with the plug **30.** The wand **36** can be removed after plug **30** has been inserted, thus ensuring that the annular portion **16** of the receptacle **10** is sealed. The escape of air **19** reduces the pressure in the annular portion **16** thus reducing the chance for mechanical failure of the receptacle **10,** particularly upon exposure to temperature change.

Further embodiments are illustrated with reference to Figs. **2 - 4****.** For the sake of clarity, similar parts in various embodiments of the present invention described hereinafter will be designated with the same reference numerals.

In contrast to the receptacle **10** of Fig. **1,** Fig. **2** shows a receptacle **40,** where an inner space **44** of the receptacle is sealed at the proximal end **56** of the receptacle. This configuration precludes the option of flowing a heat transfer fluid in via one end of the inner space **44** and out the other (sealed) end. However, this may result in a somewhat sturdier receptacle. Still, the heat transfer fluid could flow in and back out of the distal end **42,** thereby improving heat transfer.

In Fig. **2** plug **46** is shown that is different from the plug **30** (Fig. **1**) in that it does not have a bore **32.** It should be understood that the plug **30** shown in Fig. 1 could be used with the receptacle **40** shown in Fig. **2****,** and vice versa, as well as plugs and receptacles of other designs. For example - the receptacle may be sealed by an external stopper that need not be inserted into the proximal end **26.** A plugging member could be screwed onto the receptacle according to the invention, provided that the receptacle is adapted accordingly at its proximal end **26.** In fact, the annular portion of the tube may be simply sealed with a firmly attached (e.g. glued) cover.

Fig. **3** shows the receptacle **10** associated with a generally L-shaped conduit **60** comprising a leg **62** correspondingly aligned with, and for inserting into, the inner space **22** of the receptacle. The leg **62** is designed to be received within the inner space **22** of the receptacle **10** at its proximal end **26,** such that the exit of fluid from said proximal end **26** of the inner space **22** would be only through the conduit **60.** Thus, conduit **60** may be equipped with ribs **64** for improved sealing. Extending from the leg **62** is an arm **66,** which is shown comprising a downwardly directed portion **68.**

This arrangement facilitates the cooling/heating of the liquid **18,** as a heat transfer fluid can then flow into and through the inner space **22** and out the conduit **60** via arm **66,** or vice versa, similarly to the arrangement shown in Fig. **1** wherein the plug **30** has a bore **32.**

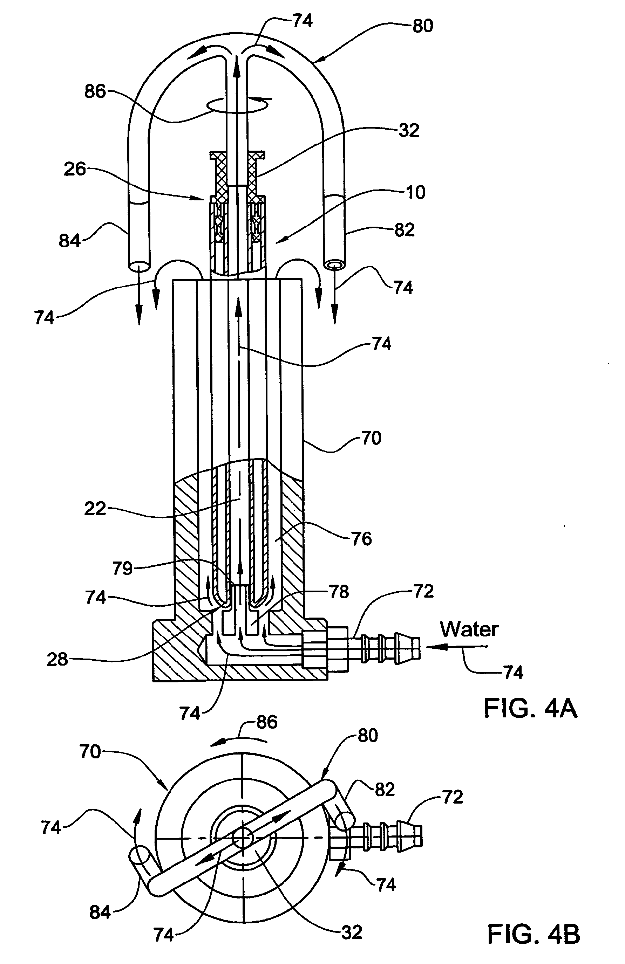

The conduit **60** is sufficient to preclude heat transfer fluid from entering the annular portion **16** without the use of a plug, however a plug is useful especially in the case where the liquid **18** is a biological liquid, or any other liquid wherein it is important to keep contaminants out, etc. The use of a plug with a bore, such as plug **30** comprising bore **32,** allows the use of a conduit in conjunction with the plug, the utility of which will be further understood from the description associated with Figs. **4A** and **4B****.**

Fig. **4A** shows a cross-section of a heat transfer chamber **70** having an inlet **72** for water or other heat transfer fluid (which can comprise a liquid, gas or mixture thereof) represented by and flowing as shown by arrows **74.** The chamber **70** further comprises a cavity **76,** designed for receiving the receptacle **10,** and, at the bottom of the cavity, a manifold **78,** for distributing the water around and through the receptacle **10.** It is appreciated that a manifold may distribute the liquid around the receptacle more evenly than would a single outlet, but the invention is not limited to having a manifold in the chamber. Additionally, there may be more than one outlet and/or inlet, and the heat transfer fluid may flow into the chamber from more than location.

The plug **30** is shown sealingly inserted in the proximal end **26** of the receptacle **10.** A conduit **80** is also shown, being a combination of a T-shape and an inverted U-shape, sealingly inserted in the bore **32** of plug **30.** The conduit **80** comprises extensions **82** and **84** that are directed in opposing directions and are perpendicular to the conduit when viewed from above (Fig. **4B**). Thus, when the water flows in the direction of arrows **74,** the receptacle **10** will spin in a direction as shown by arrow **86.** This spinning will have the effect of mixing the liquid **18** and thereby improving the uniformity of its temperature. The spinning may also improve the heat transfer rate by imparting an aspect of turbulence to the heat transfer fluid. The present invention is not limited to the shapes of conduits **60** and **80,** and conduits of countless other shapes may perform functions similar to those described.

The chamber **70** further comprises as part of manifold **78,** a protrusion **79** protruding partially into the distal end **28** of the inner space **22.** This protrusion **79** facilitates a portion of heat transfer fluid (represented by arrows **74**) to be directed into the inner space **22** and at the same time serves to position the receptacle **10** within chamber **70.**

One of the useful applications of a receptacle according to the invention is in the cryopreservation of biological samples. As discussed above, since biological samples are sensitive to freezing and thawing, it is preferred to freeze and thaw all parts of a biological sample essentially uniformly, and as close as possible to the optimal rate.

According to one aspect of the present invention, the temperature of a liquid sample in a receptacle such as receptacle **10** in Fig. **1** is changed as follows. Liquid **18** at a given temperature is first inserted into the annular portion **16** of the receptacle **10** which is then sealed with the plug **32, 46.** Alternatively, the inner space **22** is sealed with a conduit **60, 80** or by a combination of plug **32** and a conduit. The receptacle **10** containing the liquid **18** is then exposed to a temperature different from the given temperature of the sample. This is accomplished by placing the receptacle **10** in the chamber **70** and flowing a heat transfer fluid, such as water through and around the receptacle. The chamber **70** can be located in a water bath **90** (see Fig. **5**) whose temperature is controllable by any temperature control and monitoring system exemplified by system **92.** System **92** may also comprise a pump for pumping water into chamber **70** into inlet **72.**

Alternatively, and especially in the case where the liquid **18** is a biological sample, other methods for freezing the liquid sample in the receptacle **10** can be used, such as movement of the receptacle through an environment with a temperature gradient in a given direction with the longitudinal axis **20** of the receptacle being essentially parallel to that direction. Other methods are disclosed in US 5,873,254 and in co-pending PCT Application No. PCT/IL03/00026. The receptacle **10** may also be agitated during its movement along the gradient, as described in co-pending PCT Application No. PCT/IL02/00738. Alternatively, the liquid **18** can be frozen or thawed and brought to a 'final' desired temperature using the chamber **70** as just described.

### Freezing

The process of freezing a liquid sample (5% Ethylene Glycol in water) in a receptacle such as receptacle **10** having the dimensions shown in Fig. **6** was compared with a regular or "control" tube. The chamber was of the configuration shown in Fig. **4****.** The control tube was a conventional laboratory test tube, 10 cm long with an outer diameter of 1.6 cm (ASSISTENT^{™}, Germany). Both the receptacle and the control tube were made of glass.

The tube and the receptacle were each filled the liquid and two thermocouples (TCs) (ALMEMO^{R} 2290-4) were inserted into each. In the control tube the "outer" TC was situated adjacent to the outer wall of the tube and the "inner" TC was situated in the midst of the liquid. In the receptacle, the "outer" TC was situated adjacent to the outer wall of the tube and the "inner" TC was adjacent to the inner wall of the tube. The tube and receptacle were each plugged with silicon rubber stoppers such as plug **32** of Fig. **4A****.** The tube and the receptacle were each frozen by dropping into liquid nitrogen and the temperatures were measured throughout the freezing process using the TCs.

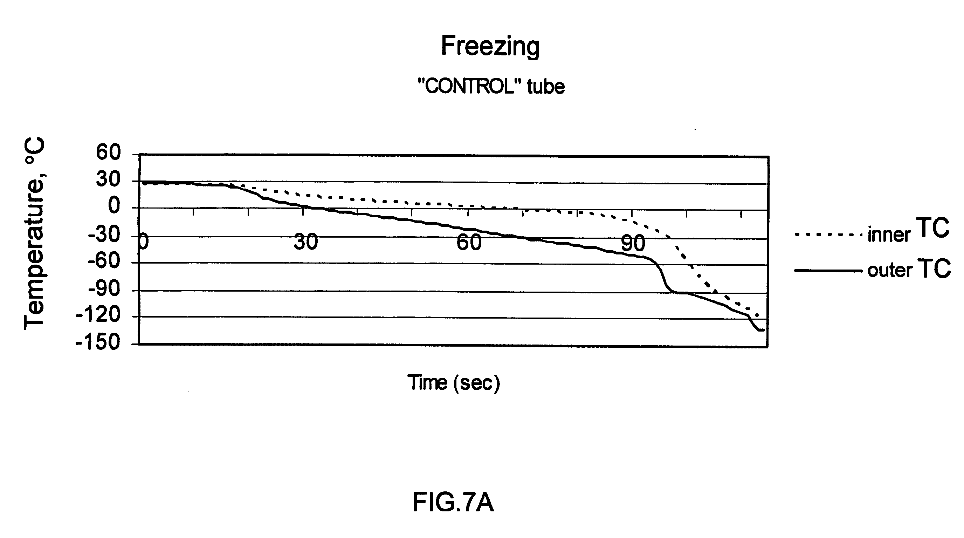

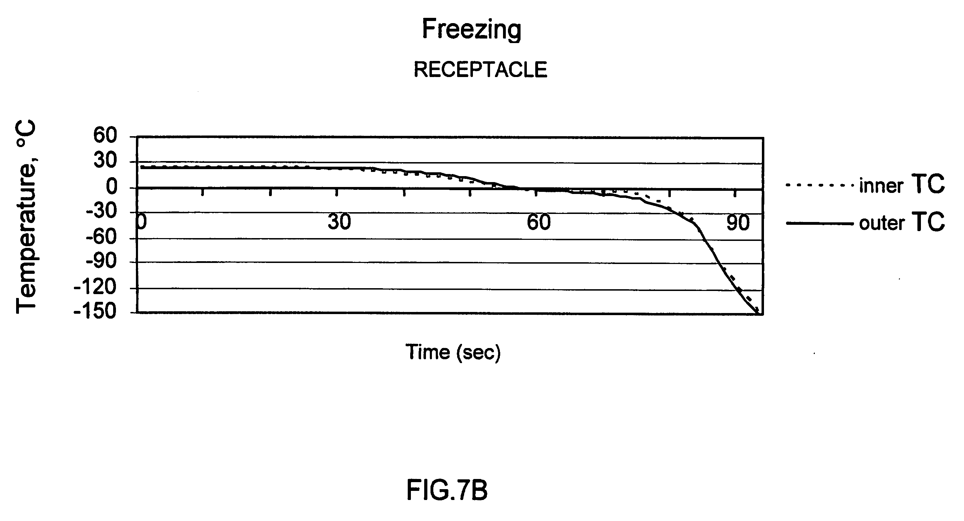

As seen in Fig. **7A****,** in the control tube there was a difference between the rate of freezing measured by the inner TC and that measured by the outer TC. In contrast, in the receptacle the rates of freezing measured by the inner and outer TCs were very similar, as seen in Fig. **7****B.**

### Thawing:

The process of thawing a liquid sample in a receptacle such as that shown in Fig. 6 was also compared with a conventional "control" tube. The tube, receptacle, liquid, plugs and TCs were the same as described above for the freezing experiment, the only difference being that just one TC was inserted into each, at a mid-point of the liquid. In the receptacle this mid-point was in the annular portion, between the inner and outer walls.

The thawing process commenced by removing both tubes from liquid nitrogen and placing at room temperature. After 1 minute and 30 seconds, when the temperature of the liquid in both cases was approximately -100 °C, the tube and the receptacle were transferred into a copper chamber (analogous to chamber **70** of Fig. **4A**) located in a conventional water bath (as shown in Fig. **5**) that was kept at a temperature of 37 °C.

The control (conventional) tube and a first receptacle were essentially motionless within the chamber during thawing, with no water flowing over the tubes. A second receptacle was inserted into the chamber, and water from the water bath was pumped through the inner space of the second receptacle at a rate of 500 ml per minute.

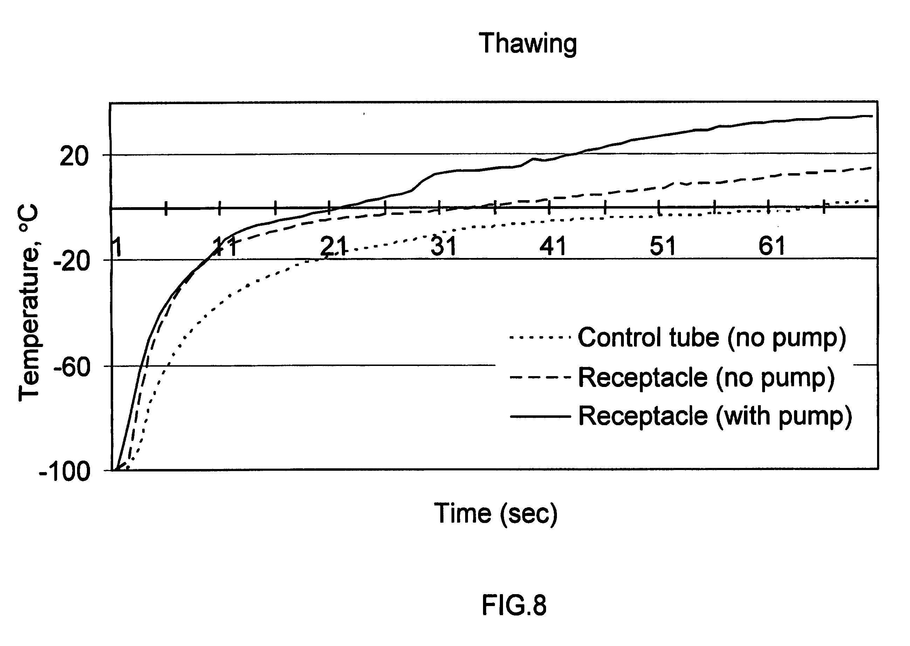

The temperatures measured during thawing in the bath are shown in Fig **8****.** The thawing rate of the first receptacle in the conventional bath was faster than that of the control (conventional) tube. The fastest thawing rate was observed with the second receptacle. This improvement was obtained in this example without rotation of the receptacle during thawing.

### Freezing and Thawing of Equine Sperm

Equine (horse) semen was frozen according to one embodiment of the present invention. A total of 97 ejaculates were collected from 31 different stallions using a Missouri model artificial vagina with an in-line filter to remove the gel fraction of semen. Each ejaculate was immediately diluted 1:1 with a conventional synthetic centrifuge medium (sorbitol extender) pre-warmed to 37°C. The solution was centrifuged at 1000xG for 25 minutes in 50ml centrifuge tubes containing 2 ml of the density medium iodixanol to reduce sperm damage and increase sperm recovery.

The resulting sperm was harvested using stainless steel cannulae, and was split into two halves. One half was diluted to 150x10⁶ sperm /ml and the other half was diluted to 60x10⁶ sperm /ml with 20% egg yolk glucose/lactose/EDTA freezing extender.

The semen diluted to 150x10⁶ sperm/ml was packaged into 0.5ml plastic straws. Semen diluted to 60x10⁶ sperm/ml was packaged into 10ml receptacles of the shape and dimensions depicted in Fig. **6**, with silicon rubber stoppers such as plug **32** of Fig. **4A****.** Straws and receptacles containing the semen were then placed into a refrigerator at 4°C and chilled for approximately 2 hours.

Straws were frozen in a conventional Planer^{™} cell freezer (pre-programmable liquid nitrogen vapor freezing device, Planer, UK and programmed with a standard equine freezing curve (IMV, France). The straws were then plunged into liquid nitrogen.

The receptacles were frozen in a model MTG516 freezing device (Interface Multigrad Technology, Israel). The initial temperature was 5 °C and the final temperature was -50°C; the receptacles being moved through the MTG516 device at a speed of 1.0 mm/sec. Seeding (freezing nucleation) was done automatically for 60 seconds. The receptacles were then plunged into liquid nitrogen.

For thawing, the straws were plunged into a water bath at 37°C for 30 seconds and then removed and dried thoroughly with paper towels. After drying the seal was cut opened at one end and the contents were transferred into a pre-warmed 4ml plastic tube.

Receptacles were thawed by removing them from the liquid nitrogen and allowing them to stand in air at room temperature for 1 minute 30 seconds. After this time they were placed into a chamber such as depicted in Figs. **4A** and **4B****,** at 37 °C for 30 seconds with water pumped at a rate of 500 ml per minute. The receptacle was removed and dried thoroughly with a paper towel. After drying, the plug was removed and a sample of semen was transferred into a pre-warmed 4ml plastic tube.

### Post-thaw evaluation:

A comparison was made between the two treatment groups by evaluating the sperm in vitro according to the following laboratory techniques:

### • Progressive Linear Motility (PLM)

Sperm were examined under phase contrast microscopy and an estimation of the percentage of sperm progressing in a substantially straight line was made and recorded. Although subjective, this test is considered the industry standard at present.

### • Osmotic resistance test (ORT)

This test partially mimics the stress exerted on sperm after insemination by incubating them in a hypotonic solution prior to staining them with a fluorescent dye that illuminates sperm intolerant of this stress. Sperm were then counted to compare a live/dead ratio.

### • AcridineOrange/Propidium Iodide dual fluorescence test (AO/PI)

Using Acridine Orange/Propidium Iodide dual fluorescence test (AO/PI) Using these two fluorescent chemicals in combination, the membrane integrity of the sperm was examined and a live/dead ratio was calculated (live sperm fluoresce green and dead sperm fluoresce red).

### • Pass/Fail criteria

Sperm that exhibited sperm motility below 35% were considered sub-standard (failing). This population would typically fail an examination and would not be used for insemination. Accordingly, sperm that exhibited sperm motility above 35% were considered to have passed.

### Results

As mentioned a total number of 97 ejaculates from 31 stallions were tested. The proportions of the sperm that passed the motility tests were 57/97 (59%) for sperm frozen in straws (Planer freeze) and 85/97 (88%) for sperm frozen in receptacles. As a control, semen from 41 ejaculates was kept unfrozen and was tested after being chilled at 5 °C for 30 hours.

Of the 97 ejaculates, only 9 (9%) produced a failing result regardless whether they were frozen in straws or receptacles (Fail-Fail group), 30 (31%) failed after having been frozen in straws but passed when frozen in receptacles (Fail-Pass group) and 55 (57%) passed regardless of whether they were frozen in a straw or receptacle (Pass-Pass group). Only 3% (3 ejaculates) failed when having been frozen in a receptacle but passed when having been frozen in straws (Pass-Fail group).

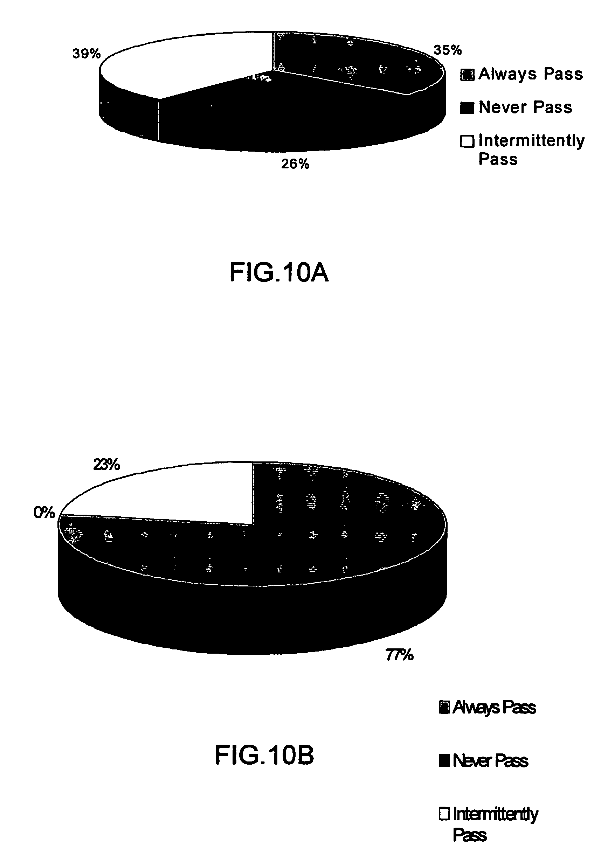

Of the 31 stallions, the following results were observed: When frozen in straws - the semen of 11 stallions (35%) always passed, 8 (26%) never passed, and 12 (39%) sometimes failed and sometimes passed (intermittently pass). These results are illustrated in a pie chart in Fig. **10A****.** When frozen in receptacles, the semen of 24 stallions (77%) always passed and 7 (23%) intermittently passed. None of the stallions' semen always failed after cryopreservation in receptacles. These results are shown in a pie chart in Fig. **10****B.** The results of the cryopreservation of the stallion semen are differently detailed in **Table I.**

**Table I**

| **Category** | **Straws** | | | **Tubes** | | |
|---|---|---|---|---|---|---|
| | **PLM (%)** | **AO/PI (% live)** | **ORT (% live)** | **PLM (%)** | **AO/PI (% live)** | **ORT (% live)** |
| **Fail-Fail (n=9)** | 19.4 | 24.2 | 15.3 | 25.0 | 32.5 | 17.8 |
| **Fail-Pass (n=30)** | 25.1 | 30.4 | 20.6 | 47.8 | 51.4 | 35.0 |
| **Pass-Pass (n=55)** | 49.6 | 49.7 | 33.2 | 58.7 | 61.8 | 42.3 |
| **Pass-Fail (n=3)** | 35.0 | 26.0 | 17.6 | 18.3 | 35.9 | 24.8 |
| ***Total (n=97)*** | **37.4** | **39.5** | **26.5** | **50.2** | **53.6** | **36.2** |

For comparison, semen that was chilled for 30 hours, gave the following average results: 55.9 % motility (n=41), 57.9 % live AO/PI (n=41) and 46.5 % live ORT (n=35). The average results of the sperm that were frozen in straws, frozen in receptacles or kept chilled without freezing are summarized in **Table II.**

**Table II**

| **Sperm treatment** | **PLM (%)** | **AO/PI (% live)** | **ORT (% live)** |
|---|---|---|---|
| **Chilled semen** | 55.9 | 57.9 | 46.5 |
| **Planer Straw (frozen)** | 37.4 | 39.5 | 26.5 |
| **Receptacle (frozen)** | 50.2 | 53.6 | 36.2 |

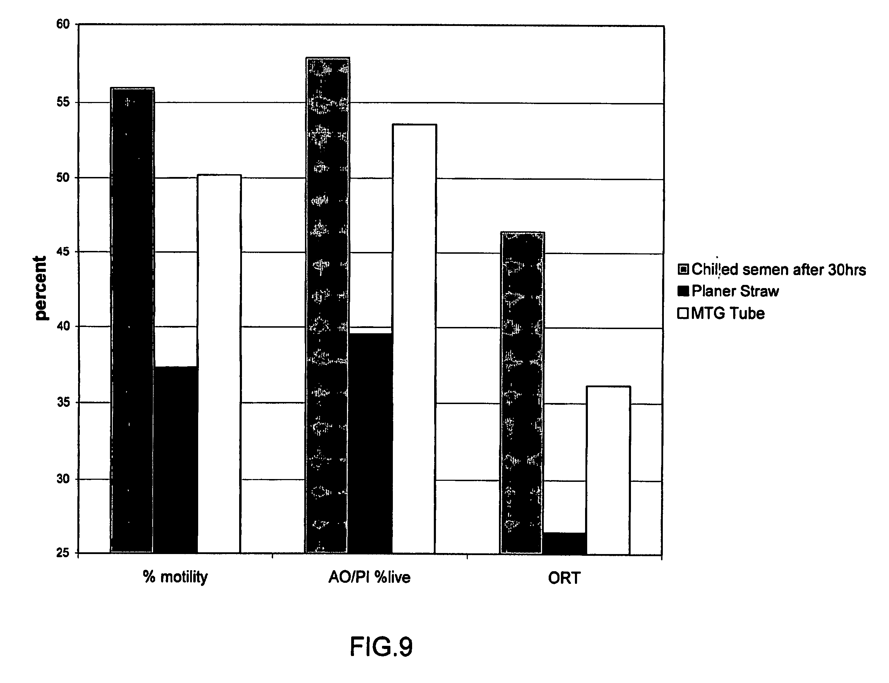

As seen in Table II and Fig. **9****,** on average, the best results were obtained by the chilled semen. However, chilled semen cannot be stored for the same duration as frozen sperm. Also seen, the sperm frozen using the receptacles displayed comparable results to those of the chilled semen both in the motility assay (PLM) and in the AO/PI staining. In all experiments the semen frozen using the receptacles gave better results than that of the straws. Some of the advantages of the present invention may include the large surface area of the receptacle for contact with heat transfer fluid - or with other heat conducting arrangements - and the improved rate and uniformity of the temperature change due to the large surface area/volume ratio.

In the sport horse breeding industry (show jumping, eventing, dressage, etc), artificial insemination using chilled semen is the preferred medium to work with over natural service. The method and apparatus of the present invention may allow the production of frozen semen that shares the benefits of chilled and conventionally frozen semen with reduced disadvantages as compared with the conventional freezing in straws.

While the above measurements and analyses were related to the freezing and thawing of equine semen, it should be understood that the results are indicative of results for many other liquid samples and the method and apparatus disclosed are applicable not only to other semen and biological samples, rather also to a variety of liquid samples wherein the controlled heating and/or cooling of a liquid is desired.

It should also be noted that the method and various components of the present invention described above, as well as variations thereof, are provided merely by way of illustration and are by no means exclusive, and many variations and modifications are possible.

For example, several alternatives may be used regarding the wand **36** to release the air **19,** which is compressed when the plug **30** or **46** is inserted into the receptacle **10** or **40.** One option is the insertion of an object such as a needle to allow the air to exit and then removal of the object to allow the plug to seal the receptacle **10** or **40.** Alternatively, the plug may comprise, for example, one or more channels for performing same with diameters and/or tortuous paths so as to prevent contamination. Also, the ribs **34** that are first inserted into the receptacle **10** or **40** may in themselves have rigid projections to allow the air to exit while the plug **30 46** is inserted. In such case the last rings **34** would then seal the receptacle **10** or **40,** as the plug is further inserted. In another example, the plug **30** or **46** could comprise ribs (or concave annular channels) on an inner wall thereof (where the plug's inner wall contacts the outer surface of the receptacle's inner wall **14**) in order to ensure sealing of the annular portion **16.**

In still another example, the conduit **60** or **80** can be of various configurations in addition to T, U, L-shaped, etc., including variations on those; and the extensions **82, 84** can be directed at various angles, although for stability during spinning it is preferable that the extensions be arranged in symmetrically opposing directions. These extensions may be fixed or fixable to the conduit **60** or **80** and in fact, the conduit **60** or **80** may in some configurations be an integral part of the receptacle **10** or **40** or the plug **30** or **46.**

In yet another example, the chamber **70** may further comprise nodules fixed to the walls of the cavity **76** to position the receptacle therein in a manner to ensure that heat transfer fluid flows uniformly around the outside of the receptacle **10** or **40.**

It is thus appreciated that the above descriptions are intended only to serve as examples and many other embodiments are possible within the scope of the claims.

## Claims

1. A receptacle (10,40) having proximal and distal ends (26,28), comprising:
- an inner wall (14) and an outer wall (12) defining an annular portion (16) therebetween adapted for receiving liquid therein, ,
- an inner space (22) defmed by said inner wall (14) open at each of said proximal and distal ends (26,28), enabling passage of fluid via said inner space (22) whilst holding said liquid within said annular portion (16), and
- a bottom wall extending between the inner and outer walls (14,12) and sealing the distal end (28) of the annular portion (16), **characterized in that** the receptacle further comprises that the proximal end (26) of the annular portion (16) is open to allow the annular portion (16) to receive said liquid therethrough.

2. The receptacle (10,40) according to claim 1, further comprising a conduit (60,80), adapted for insertion into the inner space so as to allow the fluid to exit therefrom via said conduit (60,80).

3. The receptacle (10,40) according to claim 2, wherein said conduit (60,80) is configured such that the flowing of a fluid there through imparts rotation to said receptacle (10,40).

4. The receptacle according to any one of claims 1-3, having a longitudinal axis, and
(i) the inner wall (14) defmes the inner space (22) extending along said longitudinal axis; or
**(ii)** the receptacle has a longitudinal axis and a substantially round cross-section taken perpendicularly to the longitudinal axis.

5. Use of a receptacle (10,40) as claimed in claim 1 for changing the temperature of a biological sample, said sample being **characterized by** a cross-sectional dimension along which the change of temperature may be performed with a predetermined acceptable resultant quality of the sample, said annular portion (16) of said receptacle (10,40) having a distance between said inner wall (14) and said outer wall (12) not exceeding said cross-sectional dimension of the sample.

6. A method of changing the temperature of a liquid sample, comprising:
**(i)** providing a receptacle (10,40)according to Claim 1,
**(ii)** inserting said liquid sample, at a first temperature, into said annular portion (16), and
**(iii)** exposing said receptacle (10,40) to a second temperature different from said first temperature.

7. The method according to claim **6,** wherein the liquid is a biological sample or a biological sample comprising sperm.

8. The method according to any one of claims **6-7,** wherein the receptacle (10,40) has a longitudinal axis, the inner space (22) extending along said longitudinal axis, and a substantially round cross-section taken perpendicularly to said longitudinal axis.

9. The method according to claim **8,** further comprising providing an environment with a temperature gradient in a given direction and wherein step **(iii)** is performed at least partially by passing the receptacle (10,40) in said environment along said direction, with the longitudinal axis of the receptacle (10,40) being parallel to said direction.

10. The method according to any one of claims **8** and **9,** wherein the annular portion (16) of the receptacle (10,40) is sealed at the distal end (28) and sealable at the proximal end (26) of the receptacle (10,40).

11. The method according to claim **10,** wherein the annular portion (16) is sealable at the proximal end (26) of the receptacle (10,40) by a plug (30,46) made of a resilient material and having a corresponding annular sealing portion (31), having a bore (32) surrounded by the annular sealing portion (31) and said bore (32) is adapted to be aligned with the inner space (22) of the receptacle (10,40) providing a passage thereto.

12. The method according to any one of claims **10** and **11,** wherein the method further comprises after step (ii), sealing the annular portion (16) at the proximal end (26) of the receptacle (10,40) with the annular sealing portion (31) of the plug (30,46) and wherein there is a wand (36) associated with the plug (30,46), and the step of sealing the annular portion (16) of the receptacle (10,40) at the proximal end (26) thereof is performed by:
(a) inserting the plug (30,46) into the proximal end (26) of the receptacle (10,40) so that a portion of said wand (36) enters the annular portion (16) of the receptacle (10,40) together with said annular sealing portion (31) of the plug (30,46), whereby sealing of the annular portion (16) of the receptacle (10,40) is prevented in the area of contact of the wand (36) with one of the inner or outer walls (14,12) of the receptacle (10,40), and
(b) removing the wand (36) such that the proximal end (26) of the annular portion (16) of the receptacle (10,40) becomes fully sealed.

13. The method according to any one of claims **6-12,** wherein step **(iii)** comprises providing a heat transfer fluid at the second temperature for flowing around the receptacle (10,40) and into the inner space (22) via one of the proximal or distal ends (26,28) and out of the other.

14. The method according to claim **13** further comprising, before step **(iii):**
**(I)** providing a conduit (60,80) tightly insertable in the inner space (22) of the receptacle (10,40) at the proximal end (26) thereof, being adapted to direct the heat transfer fluid flowing out of the inner space (22) so as not to enter the annular portion (16), and
**(II)** inserting said conduit (60,80) into the inner space (22) at said proximal end (26).

15. The method according to claim **14,** wherein the conduit (60,80) is configured, such that the flowing of the heat transfer fluid there through imparts rotation to the receptacle (10,40).

16. A chamber (70) containing the receptacle (10,40) according to claim 1, said chamber facilitating changing the temperature of a liquid sample held within said receptacle (10,40), and said chamber (70) comprising:
- a cavity (76) for receiving said receptacle (10,40), and
- an inlet for inputting a heat transfer fluid into said cavity (76).

17. The receptacle (10,40) according to claim **16,** further comprising a manifold (78) for distributing the heat transfer fluid through the inner space (22) and around said receptacle (10,40).

18. The receptacle according to Claim **16,** wherein the cavity (76) of the chamber (70) is adapted to accommodate the distal end (28) of the receptacle (10,40), and the manifold (78) further comprises a projection adapted to be received into the inner space (22) at the distal end (28) of said receptacle (10,40).

## Patentansprüche

1. Gefäß (10,40) mit einem proximalen und einem distalen Ende (26,28), das Folgendes umfasst:
- eine Innenwand (14) und eine Außenwand (12), die einen ringförmigen Abschnitt (16) dazwischen definieren, der zum Aufnehmen von Flüssigkeit darin angepasst ist,
- einen durch die genannte Innenwand (14) definierten Innenraum (22), der an dem proximalen und dem distalen Ende (26,28) jeweils offen ist, wodurch der Durchgang von Fluid über den genannten Innenraum (22) ermöglicht wird, während die genannte Flüssigkeit in dem genannten ringförmigen Abschnitt (16) gehalten wird, und
- eine untere Wand, die sich zwischen der Innenwand und der Außenwand (14,12) erstreckt und das distale Ende (28) des ringförmigen Abschnitts (16) verschließt,
**dadurch gekennzeichnet, dass** das Gefäß ferner umfasst, dass das proximale Ende (26) des ringförmigen Abschnitts (16) offen ist, um zuzulassen, dass der ringförmige Abschnitt (16) die genannte Flüssigkeit dadurch hindurch aufnimmt.

2. Gefäß (10,40) nach Anspruch 1, weiter umfassend eine Leitung (60,80), die zum Einfügen in den Innenraum angepasst ist, um zuzulassen, dass das Fluid über die genannte Leitung (60,80) daraus hinaus austritt.

3. Gefäß (10,40) nach Anspruch 2, wobei die genannte Leitung (60,80) derart ausgebildet ist, dass das Fließen eines Fluids dadurch hindurch dem genannten Gefäß (10,40) Drehung verleiht.

4. Gefäß nach einem der Ansprüche 1-3 mit einer Längsachse, und
(i) wobei die Innenwand (14) den Innenraum (22) definiert, der sich entlang der genannten Längsachse erstreckt; oder
(ii) wobei das Gefäß eine Längsachse und einen senkrecht zu der Längsachse im Wesentlichen runden Querschnitt aufweist.

5. Verwendung eines Gefäßes (10,40) nach Anspruch 1 zum Ändern der Temperatur einer biologischen Probe, wobei die genannte Probe **gekennzeichnet ist durch** ein Querschnittsmaß, entlang dessen die Änderung der Temperatur mit einer vorherbestimmten zulässigen resultierenden Qualität der Probe ausgeführt werden kann, wobei der genannte ringförmige Abschnitt (16) des genannten Gefäßes (10,40) einen Abstand zwischen der genannten Innenwand (14) und der genannten Außenwand (12) aufweist, der das genannte Querschnittsmaß der Probe nicht übersteigt.

6. Verfahren zum Ändern der Temperatur einer Flüssigkeitsprobe, das Folgendes umfasst:
(i) Bereitstellen eines Gefäßes (10,40) nach Anspruch 1,
(ii) Einfügen der genannten Flüssigkeitsprobe mit einer ersten Temperatur in den genannten ringförmigen Abschnitt (16), und
(iii) Aussetzen des genannten Gefäßes (10,40) einer zweiten Temperatur, die von der genannten ersten Temperatur verschieden ist.

7. Verfahren nach Anspruch 6, wobei es sich bei der Flüssigkeit um eine biologische Probe oder um eine Sperma umfassende biologische Probe handelt.

8. Verfahren nach einem der Ansprüche 6-7, wobei das Gefäß (10,40) eine Längsachse, wobei sich der Innenraum (22) entlang der genannten Längsachse erstreckt, und senkrecht zu der genannten Längsachse einen im Wesentlichen runden Querschnitt aufweist.

9. Verfahren nach Anspruch 8, weiter umfassend das Bereitstellen einer Umgebung mit einem Temperaturgradienten in einer gegebenen Richtung und wobei Schritt (iii) mindestens teilweise durch Führen des Gefäßes (10,40) in der genannten Umgebung entlang der genannten Richtung ausgeführt wird, wobei die Längsachse des Gefäßes (10,40) parallel zu der genannten Richtung ist.

10. Verfahren nach einem der Ansprüche 8 und 9, wobei der ringförmige Abschnitt (16) des Gefäßes (10,40) am distalen Ende (28) verschlossen und am proximalen Ende (26) des Gefäßes (10,40) verschließbar ist.

11. Verfahren nach Anspruch 10, wobei der ringförmige Abschnitt (16) am proximalen Ende (26) des Gefäßes (10,40) durch einen Stopfen (30,46) verschließbar ist, der aus einem elastischen Material hergestellt ist, einen entsprechenden ringförmigen Verschlussabschnitt (31) aufweist und eine Bohrung (32) aufweist, die von dem ringförmigen Verschlussabschnitt (31) umgeben ist und wobei die genannte Bohrung (32) dazu angepasst ist, mit dem Innenraum (22) des Gefäßes (10,40) zu fluchten, so dass ein Durchgang dazu hin bereitgestellt wird.

12. Verfahren nach einem der Ansprüche 10 und 11, wobei das Verfahren weiter nach Schritt (ii) das Verschließen des ringförmigen Abschnitts (16) an dem proximalen Ende (26) des Gefäßes (10,40) mit dem ringförmigen Verschlussabschnitt (31) des Stopfens (30,46) umfasst und wobei ein Stab (36) mit den Stopfen (30,46) assoziiert ist, und der Schritt des Verschließens des ringförmigen Abschnitts (16) des Gefäßes (10,40) an dem proximalen Ende (26) desselben ausgeführt wird durch:
(a) Einfügen des Stopfens (30,46) in das proximale Ende (26) des Gefäßes (10,40), so dass ein Abschnitt des genannten Stabs (36) zusammen mit dem genannten ringförmigen Verschlussabschnitt (31) des Stopfens (30,46) in den ringförmigen Abschnitt (16) des Gefäßes (10,40) eindringt, wodurch das Verschließen des ringförmigen Abschnitts (16) des Gefäßes (10,40) im Bereich des Kontakts des Stabs (36) mit der Innen- oder der Außenwand (14,12) des Gefäßes (10,40) verhindert wird, und
(b) Herausnehmen des Stabs (36), so dass das proximale Ende (26) des ringförmigen Abschnitts (16) des Gefäßes (10,40) vollständig verschlossen wird.

13. Verfahren nach einem der Ansprüche 6-12, wobei Schritt (iii) das Bereitstellen eines Wärmeübertragungsfluids mit der zweiten Temperatur zum Fließen um das Gefäß (10,40) und in den Innenraum (22) über das proximale oder das distale Ende (26,28) und aus dem anderen heraus umfasst.

14. Verfahren nach Anspruch 13, das weiter vor Schritt (iii) Folgendes umfasst:
(I) Bereitstellen einer Leitung (60,80), die an dem proximalen Ende (26) desselben fest in den Innenraum (22) des Gefäßes (10,40) eingefügt werden kann und dazu angepasst ist, das aus dem Innenraum (22) herausfließende Wärmeübertragungsfluid so zu leiten, dass es nicht in den ringförmigen Abschnitt (16) eindringt, und
(II) Einfügen der genannten Leitung (60,80) in den Innenraum (22) an dem genannten proximalen Ende (26).

15. Verfahren nach Anspruch 14, wobei die Leitung (60,80) derart ausgebildet ist, dass das Fließen des Wärmeübertragungsfluids dadurch hindurch dem Gefäß (10,40) Drehung verleiht.

16. Kammer (70), die das Gefäß (10,40) nach Anspruch 1 enthält, wobei die genannte Kammer das Ändern der Temperatur einer in dem genannten Gefäß (10,40) enthaltenen Flüssigkeitsprobe ermöglicht, und wobei die genannte Kammer (70) Folgendes umfasst:
- eine Aushöhlung (76) zum Aufnehmen des genannten Gefäßes (10,40), und
- einen Einlass zum Einfüllen eines Wärmeübertragungsfluids in die genannte Aushöhlung (76).

17. Gefäß (10,40) nach Anspruch 16, weiter umfassend einen Verteiler (78) zum Verteilen des Wärmeübertragungsfluids durch den Innenraum (22) und um das genannte Gefäß (10,40).

18. Gefäß nach Anspruch 16, wobei die Aushöhlung (76) der Kammer (70) dazu angepasst ist, das distale Ende (28) des Gefäßes (10,40) unterzubringen und der Verteiler (78) weiter einen Vorsprung umfasst, der dazu angepasst ist, an dem distalen Ende (28) des genannten Gefäßes (10,40) in den Innenraum (22) aufgenommen zu werden.

## Revendications

1. Réceptacle (10, 40) ayant des extrémités proximale et distale (26, 28), comportant :
- une paroi intérieure (14) et une paroi extérieure (12) définissant une partie annulaire (16) entre celles-ci adaptée pour recevoir du liquide dans celle-ci,
- un espace intérieur (22) défini par ladite paroi intérieure (14) ouvert à chacune desdites extrémités proximale et distale (26, 28), permettant le passage de fluide par le biais dudit espace intérieur (22) tout en retenant ledit liquide à l'intérieur de ladite partie annulaire (16), et
- une paroi inférieure s'étendant entre les parois intérieure et extérieure (14, 12) et scellant l'extrémité distale (28) de la partie annulaire (16),
**caractérisé en ce que** le réceptacle comporte par ailleurs
que l'extrémité proximale (26) de la partie annulaire (16) est ouverte pour permettre à la partie annulaire (16) de recevoir ledit liquide au travers de celle-ci.

2. Réceptacle (10, 40) selon la revendication 1, comportant par ailleurs un conduit (60, 80), adapté à des fins d'insertion dans l'espace intérieur de manière à permettre au fluide de sortir de celui-ci par le biais dudit conduit (60, 80).

3. Réceptacle (10, 40) selon la revendication 2, dans lequel ledit conduit (60, 80) est configuré de sorte que l'écoulement d'un fluide au travers de celui-ci imprime un mouvement de rotation audit réceptacle (10, 40).

4. Réceptacle selon l'une quelconque des revendications 1 à 3, ayant un axe longitudinal, et
(i) la paroi intérieure (14) définit l'espace intérieur (22) s'étendant le long dudit axe longitudinal ; ou
(ii) le réceptacle a un axe longitudinal et une section transversale sensiblement circulaire prise de manière perpendiculaire par rapport à l'axe longitudinal.

5. Utilisation d'un réceptacle (10, 40) selon la revendication 1, permettant de changer la température d'un échantillon biologique, ledit échantillon étant **caractérisé par** une dimension en coupe transversale le long de laquelle le changement de température peut être effectué avec une qualité résultante acceptable prédéterminée de l'échantillon, ladite partie annulaire (16) dudit réceptacle (10, 40) ayant une distance entre ladite paroi intérieure (14) et ladite paroi extérieure (12) ne dépassant pas ladite dimension en coupe transversale de l'échantillon.

6. Procédé permettant de changer la température d'un échantillon liquide, comportant les étapes consistant à :
(i) mettre en oeuvre un réceptacle (10, 40) selon la revendication 1,
(ii) insérer ledit échantillon liquide, à une première température, dans ladite partie annulaire (16), et
(iii) exposer ledit réceptacle (10, 40) à une seconde température différente de ladite première température.

7. Procédé selon la revendication 6, dans lequel le liquide est un échantillon biologique ou un échantillon biologique comportant du sperme.

8. Procédé selon l'une quelconque des revendications 6-7, dans lequel le réceptacle (10, 40) a un axe longitudinal, l'espace intérieur (22) s'étendant le long dudit axe longitudinal, et une section transversale sensiblement circulaire prise de manière perpendiculaire par rapport audit axe longitudinal.

9. Procédé selon la revendication 8, comportant par ailleurs la mise en oeuvre d'un environnement ayant un gradient de température dans une direction donnée et dans lequel l'étape (iii) est effectuée au moins partiellement en faisant passer le réceptacle (10, 40) dans ledit environnement le long de ladite direction, l'axe longitudinal du réceptacle (10, 40) étant parallèle à ladite direction.

10. Procédé selon l'une quelconque des revendications 8 et 9, dans lequel la partie annulaire (16) du réceptacle (10, 40) est scellée au niveau de l'extrémité distale (28) et est en mesure d'être scellée au niveau de l'extrémité proximale (26) du réceptacle (10, 40).

11. Procédé selon la revendication 10, dans lequel la partie annulaire (16) est en mesure d'être scellée au niveau de l'extrémité proximale (26) du réceptacle (10, 40) par un bouchon (30, 46) réalisé en un matériau élastique et ayant une partie de scellement annulaire correspondante (31), ayant un alésage (32) entouré par la partie de scellement annulaire (31) et ledit alésage (32) est adapté pour être aligné sur l'espace intérieur (22) du réceptacle (10, 40) à des fins de mise en oeuvre d'un passage vers celui-ci.

12. Procédé selon l'une quelconque des revendications 10 et 11, dans lequel le procédé comporte par ailleurs après l'étape (ii), l'étape consistant à sceller la partie annulaire (16) au niveau de l'extrémité proximale (26) du réceptacle (10, 40) à l'aide de la partie de scellement annulaire (31) du bouchon (30, 46) et dans lequel il y a une pipette (36) associée au bouchon (30, 46), et l'étape consistant à sceller la partie annulaire (16) du réceptacle (10, 40) au niveau de l'extrémité proximale (26) de celui-ci est effectuée par les étapes consistant à :
(a) insérer le bouchon (30, 46) dans l'extrémité proximale (26) du réceptacle (10, 40) de sorte qu'une partie de ladite pipette (36) entre dans la partie annulaire (16) du réceptacle (10, 40) en même temps que ladite partie de scellement annulaire (31) du bouchon (30, 46), ce par quoi le scellement de la partie annulaire (16) du réceptacle (10, 40) est empêché dans la zone de contact de la pipette (36) avec l'une des parois intérieure ou extérieure (14, 12) du réceptacle (10, 40), et
(b) retirer la pipette (36) de sorte que l'extrémité proximale (26) de la partie annulaire (16) du réceptacle (10, 40) devient entièrement scellée.

13. Procédé selon l'une quelconque des revendications 6 à 12, dans lequel l'étape (iii) comporte la mise en oeuvre d'un fluide de transfert de chaleur à la seconde température à des fins d'écoulement autour du réceptacle (10, 40) et dans l'espace intérieur (22) par le biais d'une des extrémités proximale ou distale (26, 28) et pour sortir de l'autre.

14. Procédé selon la revendication 13, comportant par ailleurs, avant l'étape (iii), les étapes consistant à :
(I) mettre en oeuvre un conduit (60, 80) en mesure d'être inséré de manière serrée dans l'espace intérieur (22) du réceptacle (10, 40) au niveau de l'extrémité proximale (26) de celui-ci, adapté pour diriger le fluide de transfert de chaleur s'écoulant hors de l'espace intérieur (22) de manière à ne pas entrer dans la partie annulaire (16), et
(II) insérer ledit conduit (60, 80) dans l'espace intérieur (22) au niveau de ladite extrémité proximale (26).

15. Procédé selon la revendication 14, dans lequel le conduit (60, 80) est configuré, de sorte que l'écoulement du fluide de transfert de chaleur au travers de celui-ci imprime un mouvement de rotation audit réceptacle (10, 40).

16. Chambre (70) contenant le réceptacle (10, 40) selon la revendication 1, ladite chambre facilitant l'étape consistant à changer la température d'un échantillon liquide se trouvant à l'intérieur dudit réceptacle (10, 40), et ladite chambre (70) comportant :
- une cavité (76) à des fins de réception dudit réceptacle (10, 40), et
- une entrée pour faire entrer un fluide de transfert de chaleur dans ladite cavité (76).

17. Réceptacle (10, 40) selon la revendication 16, comportant par ailleurs un collecteur (78) à des fins de distribution du fluide de transfert de chaleur dans l'espace intérieur (22) et autour dudit réceptacle (10, 40).

18. Réceptacle selon la revendication 16, dans lequel la cavité (76) de la chambre (70) est adaptée à des fins de logement de l'extrémité distale (28) du réceptacle (10, 40), et le collecteur (78) comporte par ailleurs une partie saillante adaptée à des fins de réception dans l'espace intérieur (22) au niveau de l'extrémité distale (28) dudit réceptacle (10, 40).
